# EUROPEAN PATENT SPECIFICATION

(11) **EP 2 133 062 B1**
(45) Date of publication and mention of the grant of the patent: **01.05.2013**
(21) Application number: 09251533.7
(22) Date of filing: 11.06.2009
(51) Int. Cl.: A61J 13/00, A61F 13/14

(54) **Nipple shield**
Brustwarzeschutz
Tétine protective

(30) Priority: 13.06.2008 JP 2008155727
(43) Date of publication of application: 16.12.2009
(73) Proprietor: Pigeon Corporation, Chuo-ku Tokyo 103-8480 (JP)
(72) Inventor: Yamashita, Daisuke, Tokyo 103-8480 (JP)
(74) Representative: Kremer, Simon Mark

(56) References cited:
- EP-A1- 0 442 758
- EP-A1- 1 197 198
- GB-A- 2 357 434
- US-A- 3 840 012
- US-A- 4 270 538
- US-A- 5 100 406
- US-A1- 2003 073 930

## Description

### BACKGROUND OF THE INVENTION

### 1. Field of the Invention

The present invention relates to improvements in a nipple protector for covering and protecting a woman's nipple area.

### 2. Description of the Related Art

Mothers in the lactation period, for example, suffer damage or soreness of the nipple area due to biting by the infant during nursing, and the like.

If there are problems of this kind in the nipple area, then the flow of breast milk during nursing is impaired by stress caused by pain, and as a consequence the infant sucks repeatedly with greater force in an attempt to extract a greater amount of breast milk, the nursing time becomes longer, and therefore the damage to the nipple area becomes even worse.

Therefore, nipple protectors are used in order to cover and protect the nipple area when the woman is not nursing.

EP0442758A1 describes a "breast shield" having a dome-shaped shell with an open bottom and an elastic member that extends across the open bottom. The elastic member has an opening through which the breast nipple extends into the interior of the shell. The elastic member serves as a base or mount for the shell which resiliently spaces the shell from contacting the nipple. One embodiment of the shield aids in reinverting a nipple that is inverted.

US4270538A describes a two-piece breast shield to be worn by a nursing woman. A base, contoured to confront the wearer's breast, has an-aperture through which the nipple can pass. A dome-shaped cover overlies the base, fitting onto it to define an interior milk-receiving chamber. A small vent hole near the edge of the cover is surrounded by an inwardly extending tube.

EP1197198A1 describes a breast shell comprising an outer part and an inner part, the inner part being intended to be placed on a breast. The inner part has a central orifice for the passage of the nipple within and is removably attached to the outer part so that the two parts define a hollow space. The inner part is made of a material more flexible than the outer part.

GB2357434A describes a breast shield comprising a rigid cup-shaped shell with an elastic membrane stretched across its open end having a central hole therein to receive a woman's nipple. The section of the membrane around the central hole is preferably frusto-conical and has spacers moulded thereon around the hole to space the membrane from the woman's breast. The central hole spaces the end of the woman's nipple from the inside of the shell.

US 2003/073930 A1 describes a method for preventing or healing injured nipples or areolas in mammalian females. The method involves positioning an absorbent medium over at least a portion of a nipple and areolar region of a mammalian female breast. Next, pressure is applied to the absorbent medium over at least a portion of the nipple and areolar region sufficient to prevent or heal injured nipples or areolas.

US 5100406 A describes a liner for a breast pump having a bell-shaped, flexible body which flares outwardly at one end to a size sufficient to fit over the female breast and narrows at the opposite end to an opening of a size sufficient to permit the flow of milk and which is provided with a circular extension to be connected to the pumping head. The body is provided on its outer surface with recesses providing a membranous wall portion thinner than the body.

US 3840012 A describes a two-piece device for protecting the breast nipple of a nursing woman and for collecting seeping milk. The breast contacting component has a central opening for the nipple and a cover is provided to form a hollow interior compartment where the excess milk is stored.

As a nipple protector used in this kind of application, there is, for example, the composition shown in Fig. 8 (see Japanese Patent Application Publication No. 5-33202).

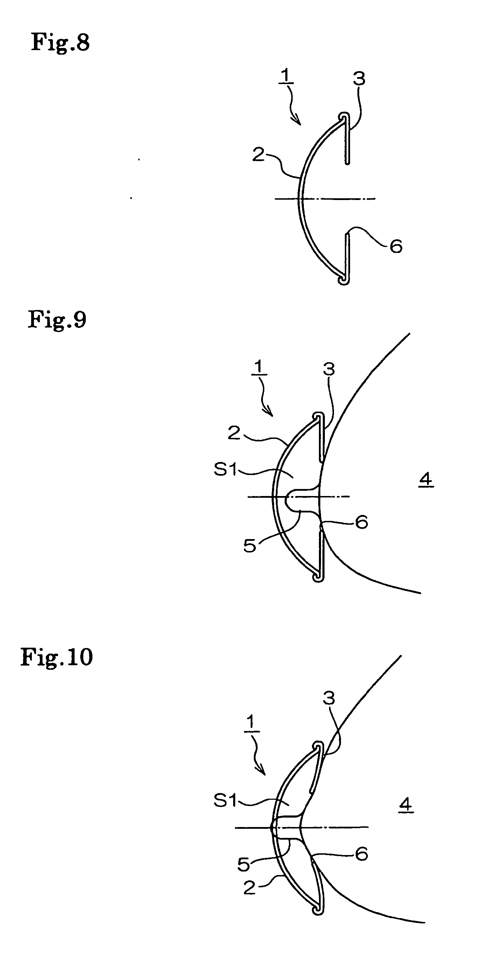

In Fig. 8, the nipple protector 1 comprises a hemispherical or dome-shaped shell 2, and an elastic member 3 which serves to close off the base portion of this shell 2.

An opening 6 is formed centrally in the elastic member 3. A plurality of air holes (not illustrated) are formed in the shell 2.

As shown in Fig. 9, the nipple protector 1 of this kind prevents any contact with the damaged nipple area 5 and prevents unwanted external irritation of the nipple area 5, by means of the elastic member 3 abutting against the front face of the breast 4 of the user, the nipple area 5 being introduced through the opening 6 in the elastic member 3 and the nipple area 5 then being accommodated in an internal space S1 which is demarcated by the elastic member 3 and the shell 2.

However, a nipple protector 1 of this kind involves problems such as the following.

This nipple protector 1 protects the nipple area 5 by accommodating the damaged nipple area 5 inside a relatively hard shell 2.

However, there is a drawback in that, when the nipple protector 1 is worn, the hard shell 2 presses against the front face of the user's breast 4 and if the hard outer edge is pressed strongly, then a circular red mark may be left on the front face of the breast 4 and the user may feel pain in the place where the edge digs into the breast.

Therefore, by disposing an elastic member 3 in a position which abuts against the front face of the breast 4 and causing this elastic member to deform in a flexible fashion, pressing of the breast 4 and unpleasant irritation of the breast 4 are eliminated.

However, as shown in Fig. 8, if the nipple protector 1 is pressed against the front face of the user's breast 4 while being worn, the elastic member 3 deforms and bends toward the inner side. Therefore, the internal space S1 formed by the shell 2 and the elastic member 3 is squashed and becomes smaller, and the inner face of the shell 2, for example, abuts directly against the nipple area 5 accommodated therein, thus causing pain and unpleasant irritation due to rubbing.

In other words, a conventional nipple protector 1 has a problem in that it is not able to display the essential function of protecting the nipple area 5 so that it does not come into contact with other objects.

### SUMMARY OF THE INVENTION

The present invention was devised in order to resolve problems such as those described above, an object thereof being to provide a nipple protector which is able to protect the nipple area of a user reliably, by protecting the nipple area so as to maintain absence of contact with the nipple area and thereby avoiding the causing of pain or unpleasant irritation to the nipple area, as well as avoiding the leaving of marks by reducing irritation when abutted against the breast.

According to a first aspect of the invention, there is provided a nipple protector (20) having an outer member (30) in a hard shell shape which forms an outer surface of the nipple protector and an inner member (40) made of a material which is more flexible and deformable than the outer member, with an internal space (S2) being formed inside the nipple protector,
wherein the internal space (S2) is formed by cylindrical portions which extend respectively from the outer member (30) and the inner member (40),
wherein the cylindrical portion extending from the outer member (30) forms a hard supporting section (33) which surrounds the perimeter of the nipple area (N) of the wearer,
and the cylindrical portion extending from the inner member (40) forms a surrounding section, which has a front end formed into an installation section (43) to abut against an inner side of the outer member (30),
and which also completely surrounds with no gap the nipple area (N), wherein the inner member comprises a deformable abutting section (45) which deforms elastically and is capable of making close contact, with no gap, with a perimeter of a nipple area (N) of a wearer,
wherein an opening (41) which connects the interior space (S2) surrounded by the surrounding section to the exterior is provided in the inner member (40), and a through hole (31) which connects to the opening (41) is provided in the outer member (30).

Since the outer member is formed from a hard material, then it is able to cover the internal space and reliably protect the nipple area (of the wearer) which is accommodated in the internal space, from external forces.

In particular, since the nipple area of the wearer, which is the object of protection, can be accommodated inside the internal space formed by a cylindrical portion extending from the outer member and/or the inner member, then the nipple area is protected in a non-contact fashion without any portion of the outer member pressing against the nipple area, and consequently, no pain or unpleasant irritation is caused to the user's nipple area.

Moreover, the deformable abutting section which abuts against the front face of the user's breast deforms upon abutting and makes close and uninterrupted contact about the perimeter or the nipple area, thus making it possible to prevent leaking of breast milk. Furthermore, since the deformable abutting section is able to make close face-to-face contact (with the front face of the breast), then compared with a case where the contact is in a line shape, or the like, the contact surface area is larger, the stimulation caused by abutment is reduced and the sealing function can be performed more reliably.

Since the cylindrical portion surrounds and protects the user's nipple area, then it is possible to maintain a non-contact state without any portion of the outer member pressing against the nipple area.

Since the surrounding section is supported by an outer member against which the surrounding section formed by the cylindrical portion abuts, then it is possible to protect the user's nipple area in a suitable fashion, while maintaining a constant volume at all times of the internal space formed by the cylindrical portion. Furthermore, since the deformable abutting section is pressed appropriately against the front face of the user's breast, then it is possible to achieve elastic contact in a reliable fashion.

Since the hard supporting section which is the cylindrical portion extending from the outer member surrounds the perimeter of the nipple area and does not deform, then it is possible to prevent even more effectively a situation where a portion of the outer member is pressed against the nipple area, and since the surrounding portion of the flexible inner member is installed on the front end portion of the hard supporting section and covers same, then the sealing properties are improved.

According to a further aspect of the invention, in the composition of the above aspect of invention, the surrounding section of the inner member has a wall section allowing no gap, and as a continuation from the wall section, a bend section which curves inwards on an inner side thereof is provided, and as continuations from the bend section, the deformable abutting section which expands outwards, and an abutting and protecting section which bends outwards on the outer side of the deformable abutting section and covers the outer edge of the outer member are provided.

Since the surrounding section of the inner member has a wall section and, formed integrally with same, a bend section which bends inwards, and therefore it is possible to make the deformable abutting section deform appropriately and abut against the front face of the user's breast, by means of the bending and deforming function of the bending section. Moreover, since there is an abutting and protecting section which bends outwards on the outside of the deformable abutting section and covers the outer edge of the outer member, then it is possible to achieve suitable protection without the outer edge section digging into the front face of the breast.

According to the composition of the first aspect of the invention, even if breast milk is secreted from the nipple area which is accommodated in the internal space, this milk passes through the opening in the inner member and exits to the exterior by passing through the through hole in the outer member, and therefore it is possible to absorb this milk by duly positioning a breast pad, or the like.

According to a yet further aspect of the invention, in the composition of the first aspect of the invention, an opening having a larger surface area than the opening or the through hole is formed in a position of the inner member on the outside of the opening and/or a position of the outer member on the outside of the through hole.

Since an opening having a larger surface area than the opening is provided in a position of the inner member to the outside of the opening and/or a position of the outer member to the outside of the through hole, then even though two or more members are used in the nipple protector formed by assembling together the inner member and the outer member, the weight is reduced correspondingly by the large opening thus formed, and therefore the overall weight is low. Consequently, when the nipple protector is in a fitted state, positional deviation is not liable to occur and the fitted state can be maintained satisfactorily.

Furthermore, it is also possible to prevent a stifling sensation caused by close contact of the full surface of the deformable abutting section.

As described above, according to the present invention, it is possible to provide a nipple protector which is able to protect the nipple area of a user reliably, by protecting the nipple area so as to maintain absence of contact with the nipple area and thereby avoiding the causing of pain or unpleasant irritation to the nipple area, as well as avoiding the leaving of marks by reducing irritation when abutted against the breast.

### BRIEF DESCRIPTION OF THE DRAWINGS

Fig. 1 is an approximate exploded perspective diagram of a nipple protector;
Fig. 2 is an approximate exploded plan diagram of the nipple protector in Fig. 1;
Fig. 3 is an approximate exploded side cross-section diagram of the nipple protector in Fig. 1;
Fig. 4 is an approximate vertical cross-section diagram showing a state where a user has fitted the nipple protector in Fig. 1;
Fig. 5 is an approximate side cross-section diagram showing the nipple protector in Fig. 1 in a use state;
Fig. 6 is an approximate exploded side cross-section diagram of a nipple protector;
Fig. 7 is an approximate vertical cross-sectional diagram showing a state where a user has fitted the nipple protector in Fig. 6;
Fig. 8 is an approximate cross-sectional diagram showing one example of a conventional nipple protector;
Fig. 9 is an approximate cross-section diagram showing the nipple protector in Fig. 8 in a use state; and
Fig. 10 is an approximate cross-section diagram showing the nipple protector in Fig. 8 in a use state.

### DESCRIPTION OF THE PREFERRED EMBODIMENTS

Below, nipple protectors are described in detail with reference to the accompanying drawings.

The embodiments described below are subject to various desirable technical restrictions, but are not limited to these modes, unless particular restrictions are stated expressly in the following description.

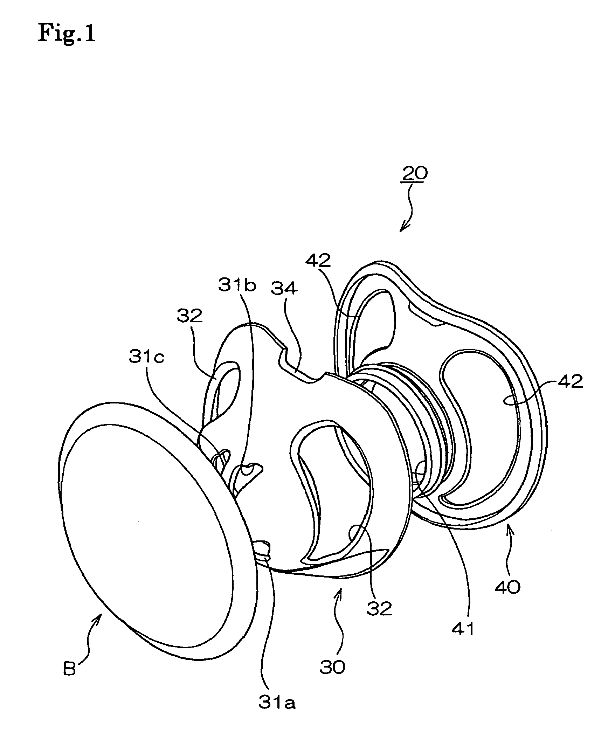

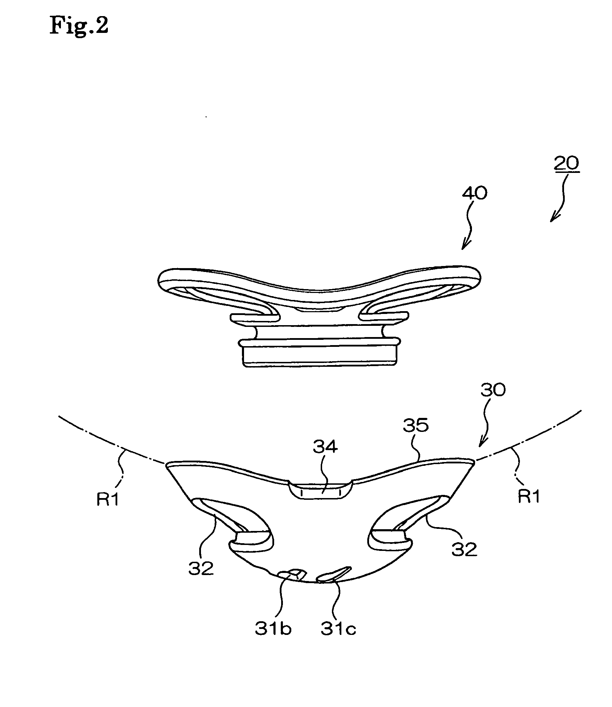

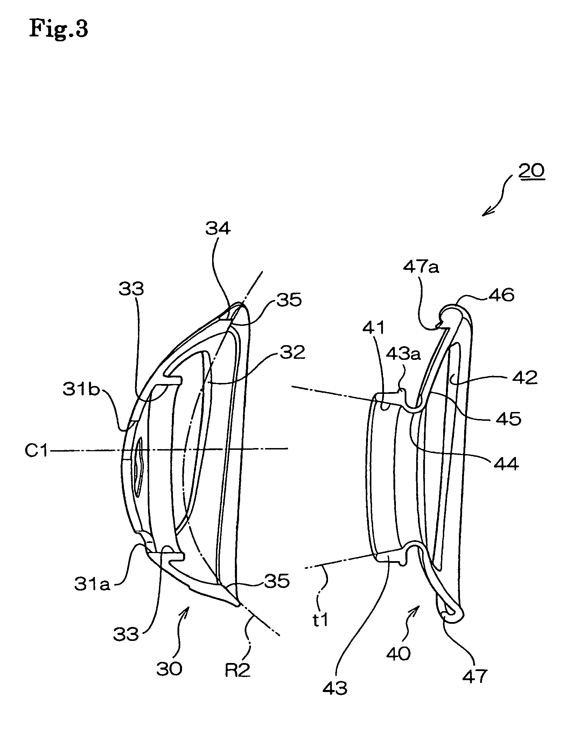

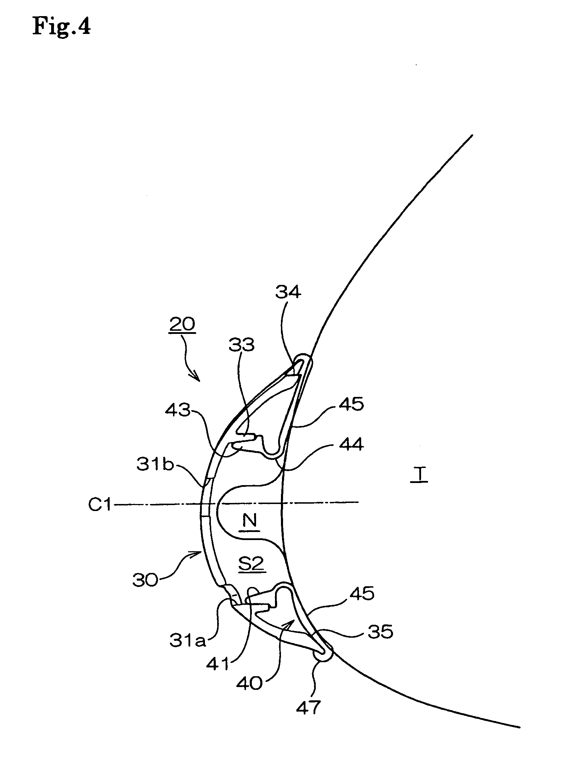

Fig. 1 is an approximate exploded perspective diagram showing a nipple protector, Fig. 2 is an approximate exploded plan view of a nipple protector (a plan view), Fig. 3 is an approximate side cross-section diagram showing an exploded state of the nipple protector, Fig. 4 is an approximate vertical cross-section diagram of a state where the nipple protector is abutted against the front face of the user.

An embodiment of a nipple protector is described in detail with reference to these drawings.

The nipple protector 20 is formed by combining an outer member 30 forming a guard portion which is a moulded member made of hard plastic having overall an approximate shell shape or an approximate dome shape or hemispherical shape, and a flexible inner member 40 forming a breast abutting portion which is installed on the inner side of the outer member (in Fig. 1, the "rear side").

As can be seen from Fig. 1, the outer shapes of the outer member 30 and the inner member 40 are desirably heart shapes which are close to a circular shape, when viewed from the front side, for example. Therefore, the outer member 30 and the inner member 40 adopt a mode which has vertical orientation, in other words, a mode where the up/down direction during installation is previously determined.

The outer member 30 is a hard plastic molding, which is made of PP (polypropylene), for example. The material of the outer member 30 is of course not limited to polypropylene. Any material which is moldable and has the necessary hardness can be used.

The outer member 30 is practically dome-shaped or hemispherical-shaped, and the inner portion thereof has a concave shape so as to form a space (see internal space S2 in Fig. 4).

Through holes which pass from the interior to the exterior are formed in approximately the central portion of the outer member 30. Desirably, a plurality of through holes are formed, and in the case shown in the drawings, the through holes indicated by reference numerals 31a, 31b and 31c are formed. Of the plurality of through holes, a portion of the through holes 31a is disposed below the virtual central line C1 of the vertical direction (height direction), as shown in Fig. 3 and Fig. 4.

Large slits or openings 32, 32 having shapes with left/right symmetry are formed respectively in the region outside the central portion of the outer member 30 where the through holes 31a, 31b and 31c are formed. If at least two of the openings 32, 32 are combined, they are provided with a larger opening surface area than the total opening surface area of the through holes 31a, 31b and 31c, and are formed in a comma-shape which avoids the central portion of the outer member 30, forming a large circular arc in the upper portion and tapering to a narrow end in the lower portion.

By forming large openings 32, 32 by removing the material of the outer member 30 in this way, it is possible to reduce the weight accordingly. Accordingly, in a state where the nipple protector 20 is installed, it is possible to prevent effectively the occurrence of deviation in the installation position due to reduction in the overall inertia. Furthermore, even when the deformable abutting section lies in close contact with the breast, it is possible to ensure passage of air by means of the openings 32, 32, and therefore it is possible to prevent a stifling sensation caused by the close contact.

Moreover, as shown in Fig. 3, a supporting section 33 is formed which is a cylindrical portion that extends inwards (toward the right-hand side in Fig. 3) from the inner side (rear side) of the outer member 30, at a position slightly to the outer side of the through holes 31a, 31b and 31c at the least. The supporting section 33 is made of a hard material which is formed in an integrated fashion with the outer member 30, and is a wall-shaped rib which stands in upright fashion in the same direction as the virtual central line C1 of the vertical direction of the outer member 30 shown in Fig. 3.

In this embodiment, the rib forming the supporting section 33 is formed in the shape of an uninterrupted wall so as to surround the region to the outside of the central region where the through holes 31a, 31b and 31c are formed. By this means, it is possible to form a space inside the supporting section 33, and this space does not deform since it is demarcated by the hard supporting section 33.

A positioning section 34 consisting of a recess, for example, is formed in the upper end position of the outer member 30. As described below, this recess 34 guides the outer member 30 to an accurate installation position, when assembled together with the inner member 40.

Furthermore, the perimeter edge section 35 of the outer member 30 is formed with a planar or curved surface of a shape that follows the curves R1 and R2 of the outer surface of the breast T, taking account of the fact that the nipple protector 20 is disposed on the front face of the breast T of the user, as shown in Fig. 2 and Fig. 4.

In other words, the perimeter edge section 35 is a planar surface or curved surface of narrow width which obliquely follows the curves R1 and R2 of the outer surface of the breast T.

Here, R1 in Fig. 2 is the curved surface produced by the shape of the breast T projecting in the forward direction, when viewed from above, and R2 in Fig. 3 represents the curved surface of same when viewed from the side.

By providing the perimeter edge section 35 of the outer member with a surface area of a prescribed width, it is possible to cause the deformable abutting section to make face to face contact with the breast in conjunction with the function of the deformable abutting section described below, when the outer member 30 is abutted against the front face of the breast T of the user via the inner member.

Next, the inner member 40 will be described.

In the nipple protector 20, as well as preventing external forces of any kind from acting on the nipple area which is accommodated in the internal space and preventing direct contact with external objects, due to the fact that the hard outer member 30 does not deform, the inner member 40 has a function of preventing the inner surface of the nipple protector 20 itself from touching the injured nipple area. On the other hand, when the nipple protector 20 is installed, the inner member 40 serves as a shock absorber that prevents the hard outer member 30 from abutting directly against the user's skin and creating unpleasant irritation.

In order to display a function of this kind, the inner member 40 is formed entirely from a soft material. For this soft material, it is possible to use silicone, for example. The hardness of the material for forming the inner member 40 is 50 as measured using a JIS (K6253) type durometer, for example. The hardness of the material varies with the thickness of the material, and the like, but desirably, the hardness is approximately 35 to 65, with a view to being able to achieve the functions and actions described below in the relation to the present embodiment, as well as obtaining shape retaining properties and formability, and the like.

For the material of the inner member 40, it is possible to use a material other than silicone which is more pliant than the outer member 30, which deforms readily by having a certain degree of rubber elasticity, and which has excellent formability.

The outer shape of the inner member 40 approximates that of the outer member 30, and their respective outer shapes are virtually superimposed.

In other words, the overall shape of the inner member 40 is a heart shape which is close to a circular shape, and comprises a cylindrical opening 41 in the central portion. The cylindrical opening 41 is a through hole. The inner side of the cylindrical opening 41 forms an internal space S2.

Large slits or openings 42, 42 forming shapes with left/right symmetry are formed respectively in the region to the outside of the central portion where the cylindrical opening 41 is formed. If at least two openings 42, 42 are aligned, they are provided with a larger opening surface area than the opening surface area of the cylindrical opening 41, and are formed in a comma-shape which avoids the central portion of the outer member 30, forming a large circular arc in the upper portion and tapering to a narrow end in the lower portion.

By forming large openings 42, 42 by removing the material in the inner member 40 in this way, it is possible to reduce the weight accordingly. Accordingly, in a state where the nipple protector 20 is installed, it is possible to prevent effectively the occurrence of deviation in the installation position due to reduction in the overall inertia. Furthermore, even when the deformable abutting section lies in close contact with the breast, it is possible to ensure passage of air by the openings 32, 32, and therefore it is possible to prevent a stifling sensation caused by the close contact.

In Fig. 3 and Fig. 4, in the inner member 40, the perimeter edge of the cylindrical opening 41 forms a low cylindrical portion which extends from the perimeter edge of the opening, and this cylindrical portion forms a surrounding section which surrounds the perimeter of the nipple area N of the user. Moreover, in this embodiment, the surrounding section forms an installation section 43 for installing the supporting section 33 of the outer member 30 on the inner member 40.

The installation section 43 is provided with an interlocking step section 43a which is a laterally-oriented step section that is attached by being fitted into the supporting section 33 formed by the wall-shaped rib of the outer member 30 and which abuts against the front end of the supporting section 33 when, on the outside of the installation section 43, fitted into the supporting section 33.

In other words, when installed, the installation section 43 covers the front end of the supporting section 33, and furthermore the cylindrical opening 41 on the inner side of the installation section 43 is tapered so as to open toward the front direction, as indicated by the broken line t1.

A bend section 44 is provided in a connected fashion to the installation section 43 of the inner member 40, and a deformable abutting section 45 which extends toward the outer circumferential direction is provided in a connected fashion to this bend section 44.

The inside portion of the bend section 44 which is the base end portion of the installation section 43 has a shape which bends to the inside, and the final end portion of the bend section 44 is a curved surface which is substantially the same as the curves R1 and R2 in Fig. 2 and Fig. 3, in other words, a curved surface which is slightly convex in the downward direction in Fig. 2 and in the leftward direction in Fig. 3, and which becomes the deformable abutting section 45 described above.

Moreover, the outer edge portion of the deformable abutting section 45 is curved outwards and forms an abutting and protecting section 46 which covers the outer edge of the outer member 30.

As can be seen from Fig. 3, the outer edge portion of the inner member 40 forms an attachment section 47 which has a groove that is open obliquely in the forward direction. The attachment section 47 enables the inner member 40 to be readily attached to and detached from the outer member 30, by means of the outer edge portion of the outer member 30 being fitted into and detached from the groove in the attachment section 47.

Moreover, an engaging section 47a which is an engaging hook projecting in the forward direction, for example, is formed on the upper end position of the inner member 40. By inserting this engaging section 47a into the positioning section 34 of the outer member 30, it is possible to achieve a correctly positioned and combined state of the outer member 30 and the inner member 40.

The present embodiment has the composition described above and can be used as follows.

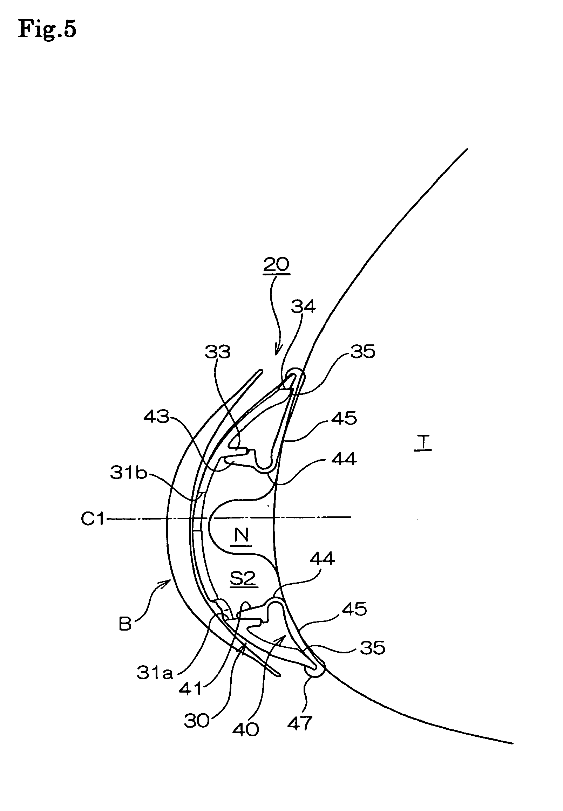

Fig. 5 shows a state where the nipple protector 20 is disposed on the front face of the breast T of a user, a breast pad B is placed against the outer side thereof, and is held in place by underwear, such as a bra (not illustrated).

In the nipple protector 20 according to the present embodiment, since the outer member 30 is formed from a hard material, then it is able to cover the internal space S2 and reliably protect the nipple area N (of the wearer) accommodated in the internal space S2 from external forces.

Here, in cases where the nipple area N accommodated in the internal space S2 is injured, then if the internal space S2 is crushed and constricted, due to the elastic deformation of the outer surface of the nipple protector 20, for instance, then the inner side (inner surface) of the nipple protector 20 makes contact with the nipple area N, thus causing unpleasant irritation, pain, and the like.

However, in the present embodiment, since the outer member 30 comprises a hard wall-shaped supporting section 33 which surrounds the perimeter of the nipple area N, then the internal space S2 does not deform and the nipple area N is protected by means of the hard supporting section 33 which surrounds the perimeter thereof. Consequently, since the nipple area N is protected in a non-contact fashion at all times, without being pressed by any portion of the outer member 30, then no pain or unpleasant irritation is caused to the user's nipple area N.

Instead of the hard supporting section 33 of the outer member 30, it is also possible to form an internal space S2 by means of a cylindrical portion extending from the inner member 40, by which means the nipple area N can also be protected.

Furthermore, since the installation section 43 of the flexible inner member 40 is formed so as to cover the front end portion of the hard supporting section 33, then the hard supporting section 33 never abuts against the user's breast T, thereby causing unpleasant irritation or pain.

Moreover, the inner member 40 has a deformable abutting section 45 which is able to make close and uninterrupted face-to-face contact with the perimeter of the nipple area N. In other words, in the inner member 40, the deformable abutting section 45 which abuts against the front face of the user's breast T deforms upon abutting and makes close and uninterrupted contact about the perimeter of the nipple area N, thereby making it possible to prevent breast milk from leaking to the exterior from the internal space S2. Moreover, since the deformable abutting section 45 is able to make close face-to-face contact (with the front face of the breast T), then compared with a case where the contact is in a line shape, or the like, the contact surface area is larger, the stimulation caused by abutment is reduced and the sealing function can be performed more reliably.

Breast milk secreted from the nipple area N passes through the cylindrical opening 41 from the internal space S2 and is then guided to the exterior from the through holes 31a, 31b and 31c of the outer member 30 which connect with the cylindrical opening 41, and is absorbed by the breast pad B.

Furthermore, the installation section 43 of the inner member 40 has a wall portion which allows no gap and a bend section 44 which curves inwards formed integrally with the wall. Therefore, due to the bending and deforming function of the bend section 44, it is possible to make the deformable abutting section 45 deform appropriately and abut against the front face of the user's breast T.

Moreover, since there is an abutting and projecting section 46 which curves outwards from the outer side of the deformable abutting section 45 and covers the outer edge of the outer member 30, then it is possible to achieve suitable protection without the outer edge portion digging into the front face of the breast T or causing unpleasant irritation.

Furthermore, the uninterrupted wall portion which forms the installation section 43 has a tapered shape which expands in diameter gradually toward the outer member 30, as indicated by reference numeral tl in Fig. 3. Consequently, it is possible effectively to prevent local stagnation of breast milk which has collected in the internal space S2 that is demarcated by the wall portion, and the discharge of the collected breast milk to the exterior is facilitated.

Accordingly, there is no risk of breast milk leaking from the internal space S2 to the side of the breast T and soiling the user's clothing, but rather, the milk is absorbed appropriately by the breast pad B.

Furthermore, by this means, the injured nipple area N remains dry, rather than being immersed in collected breast milk, and therefore the injury can heal more readily and growth of bacteria is suppressed.

Furthermore, of the plurality of through holes in the outer member 30, a portion of the through holes 31a is disposed below the virtual central line C1 in the vertical direction (height direction), as shown in Fig. 3 and Fig. 4. Therefore, all of the breast milk is readily discharged to the exterior, rather than collecting in the lower portion of the internal space S2.

Moreover, in an inner member 40 large slits or openings 42, 42 are formed in the left and right-hand outer sides of the cylindrical opening 41. Furthermore, since openings 32, 32 having a larger surface area than the total opening surface area of the through holes 31a, 31b, 31c are provided in the outer member 30 at positions to the left and right-hand outer sides of the through holes, then even though two or more members are used in the nipple protector 20 which combines an outer member 30 and the inner member 40, the weight is reduced correspondingly by the large openings thus formed, and therefore the overall weight is low. Consequently, when the nipple protector 20 is in a fitted state, positional deviation is not liable to occur and the fitted state can be maintained satisfactorily.

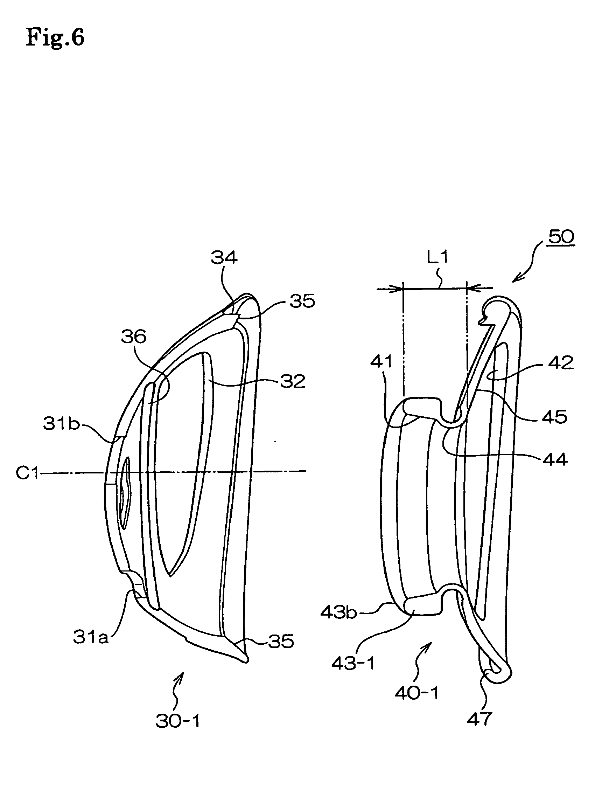

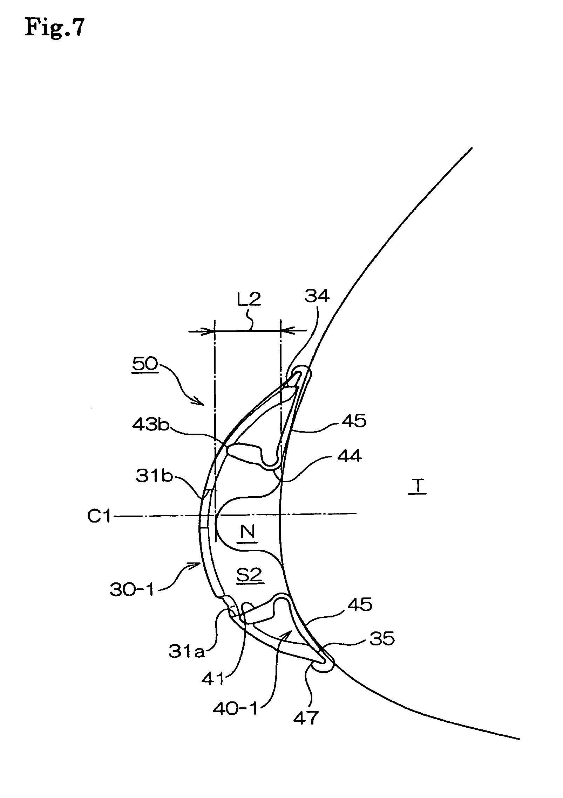

Fig. 6 and Fig. 7 show a second nipple protector , Fig. 6 is an exploded side cross-sectional view, and Fig. 7 is a diagram showing a fitted state (use state).

In these diagrams, parts of the composition which are common to the first nipple protector are labelled with the same reference numerals and repeated description thereof is omitted. The following description centers on the points of difference.

In this nipple protector, rather than forming the supporting section which is a cylindrical portion of the outer member 30-1, a cylindrical portion 43-1 is formed only on the inner member 40-1. This cylindrical portion 43-1 is a surrounding section which surrounds the perimeter of the nipple area of the user, and has the form of a thick wall which comprises a bend section 44 as well as having a sufficient height (length) L1.

In other words, desirably, as shown in Fig. 7, the cylindrical portion 43-1 has at least the same height or a greater height than the average height (length) L2 of the nipple area of the user. The cylindrical portion 43-1 is desirably composed so as to abut against the inner surface of the outer member 30-1.

Preferably, the dimension of L1 is approximately 5 mm to 20 mm.

Furthermore, desirably, a groove section 36 forming an accommodating section as shown in Fig. 6 is formed in the outer member 30-1. In other words, a portion for accommodating the front end portion 43b of the cylindrical portion 43-1, which forms a surrounding section extending from the inner member when the inner member 40-1 is assembled with the outer member 30-1 as shown in Fig. 7, is formed on the inner side of the outer member 30-1 at the abutting position of the front end portion 43b, and this accommodating section adopts the mode of a groove section 36 which is a circular groove formed in a ring shape, for example.

The cylindrical portion 43-1 extending from the inner member 40-1 surrounds and protects the nipple area N of the user, and therefore no portion of the outer member 30-1 is pressed against the nipple area N and a state of non-contact can be maintained (see Fig. 7).

Moreover, since the cylindrical portion 43-1 is supported by the outer member 30-1 against which the cylindrical portion 43-1 is abutted, then the volume of the internal space S2 formed by the cylindrical portion 43-1 remains unchanged at all times and the nipple area of the user can be protected in an appropriate fashion. Furthermore, since the deformable abutting section 45 is pressed appropriately against the front face of the user's breast T, then it is possible to achieve elastic contact in a reliable fashion.

Moreover, by providing a groove section 36, for example, to act as an accommodating section in the outer member 30-1, then the cylindrical portion 43-1 is held accurately in a prescribed position on the inner surface of the outer member 30-1, and therefore the internal space S2 is formed in an even more reliable fashion.

It should be noted that the present invention is not limited to the embodiments described above.

In the protectors described above, the cylindrical portion for forming the internal space is formed respectively as a supporting section 33 of the outer member 30 and an installation section 43 of the inner member 40, but it may also be formed in either one of the outer member or the inner member only.

Consequently, as also described above, the large opening which forms the internal space may also be formed in either one of the outer member 30 and the inner member 40. If the cylindrical portion is formed only in the outer member, then desirably the front end portion of the cylindrical portion is sufficiently covered by the inner member, so as to reduce to a minimum the irritation caused by abutment against the front face of the user's breast.

For the outer shape of the nipple protector 20, it is possible to employ various shapes other than a heart shape, such as a petal shape, a geometric shape, or the like, but desirably, a design which has vertical orientation is adopted, since this is beneficial in making it possible to assemble and install the protector without errors, if, for example, the breast pad B has a similar vertical orientation.

Apart from being formed as two separate members as described above, the inner member and the outer member may also be formed as two-colored moldings or multi-colored moldings which combine the use of materials of different types.

Furthermore, the individual compositional elements of the embodiments described may be omitted according to requirements, and combined with other compositions which are not described as defined by the claims.

## Claims

1. A nipple protector (20) having an outer member (30) in a hard shell shape which forms an outer surface of the nipple protector and an inner member (40) made of a material which is more flexible and deformable than the outer member, with an internal space (S2) being formed inside the nipple protector,
wherein the internal space (S2) is formed by cylindrical portions which extend respectively from the outer member (30) and the inner member (40),
wherein the cylindrical portion extending from the outer member (30) forms a hard supporting section (33) which surrounds the perimeter of the nipple area (N) of the wearer,
and the cylindrical portion extending from the inner member (40) forms a surrounding section, which has a front end formed into an installation section (43) to abut against an inner side of the outer member (30),
and which also completely surrounds with no gap the nipple area (N), wherein the inner member comprises a deformable abutting section (45) which deforms elastically and is capable of making close contact, with no gap, with a perimeter of a nipple area (N) of a wearer,
wherein an opening (41) which connects the interior space (S2) surrounded by the surrounding section to the exterior is provided in the inner member (40), and a through hole (31) which connects to the opening (41) is provided in the outer member (30).

2. The nipple protector (20) according to claim 1,
wherein the installation section (43) covers the front end portion of the supporting section (33).

3. The nipple protector (20) according to either one of claims 1 or 2, wherein the surrounding section of the inner member (40) has an uninterrupted wall section (43), and as a continuation from the wall section (43), a bend section (44) which curves inwards on an inner side thereof is provided, and as continuations from the bend section (44), the deformable abutting section (45) which expands outwards, and an abutting and protecting section (46) which bends outwards on an outer side of the deformable abutting section (45) and covers an outer edge of the outer member (30) are provided.

4. The nipple protector (20) according to claim 1,
wherein an opening (42) having a larger surface area than the opening (41) or the through hole (31) is formed in a position of the inner member (40) on the outside of the opening (41) and/or a position of the outer member (30) on the outside of the through hole (31).

## Patentansprüche

1. Brustwarzenschutz (20) mit einem äußeren Element (30) in einer Hartschalenform, die eine Außenoberfläche des Brustwarzenschutzes bildet, und einem inneren Element (40) aus einem Material, das flexibler und verformbarer ist als das äußere Element, mit einem Innenraum (S2), der im Inneren des Brustwarzenschutzes ausgebildet ist,
wobei der Innenraum (S2) durch zylinderförmige Abschnitte ausgebildet ist, die sich jeweils von dem äußeren Element (30) und dem inneren Element (40) erstrecken,
wobei der zylinderförmige Abschnitt, der sich von dem äußeren Element (30) erstreckt, einen harten Stützabschnitt (33) bildet, der den Umfang des Brustwarzenbereichs (N) der Trägerin umgibt,
und der zylinderförmige Abschnitt, der sich von dem inneren Element (40) erstreckt, einen umgebenden Abschnitt bildet, der ein vorderes Ende aufweist, das zu einem Befestigungsabschnitt (43) geformt ist, der an einer Innenseite des äußeren Elements (30) anliegt,
und der den Brustwarzenbereich (N) ebenfalls ohne Zwischenraum vollständig umgibt, wobei das innere Element einen verformbaren anliegenden Abschnitt (45) umfasst, der sich elastisch verformt und einen engen Kontakt ohne Zwischenraum mit dem Umfang des Brustwarzenbereichs (N) einer Trägerin herstellen kann,
wobei eine Öffnung (41), die eine Verbindung des durch den umgebenden Abschnitt umgebenen Innenraums (S2) nach außen herstellt, in dem inneren Element (40) bereitgestellt ist und ein Durchgangsloch (31), das eine Verbindung zu der Öffnung (41) herstellt, in dem äußeren Element (30) bereitgestellt ist.

2. Brustwarzenschutz (20) nach Anspruch 1, worin der Befestigungsabschnitt (43) den vorderen Endabschnitt des Stützabschnitts (33) abdeckt.

3. Brustwarzenschutz (20) nach einem der Ansprüche 1 oder 2, worin der umgebende Abschnitt des inneren Elements (40) einen durchgehenden Wandabschnitt (43) aufweist, und als Fortsetzung des Wandabschnitts (43) ein Biegungsabschnitt (44) bereitgestellt ist, der an einer Innenseite eine Krümmung nach innen aufweist, und als Fortsetzung des Biegungsabschnitts (44) der verformbare anliegende Abschnitt (45), der sich nach außen erstreckt, und ein anliegender Schutzabschnitt (46), der an einer Außenseite des verformbaren anliegenden Abschnitts (45) nach außen gebogen ist und einen Außenrand des äußeren Elements (30) abdeckt, bereitgestellt sind.

4. Brustwarzenschutz (20) nach Anspruch 1, worin eine Öffnung (42) mit einer größeren Oberfläche als die Öffnung (41) oder das Durchgangsloch (31) an einer Stelle des inneren Elements (40) an der Außenseite der Öffnung (41) und/oder an einer Stelle des äußeren Elements (30) an der Außenseite des Durchgangslochs (31) ausgebildet ist.

## Revendications

1. Cache-tétine (20) ayant un élément extérieur (30) sous une forme de coque dure qui constitue une surface extérieure du cache-tétine et un élément intérieur (40) réalisé en un matériau qui est plus flexible et déformable que l'élément extérieur, un espace intérieur (S2) étant formé à l'intérieur de cache-tétine,
où l'espace intérieur (S2) est formé par des portions cylindriques qui s'étendent respectivement depuis l'élément extérieur (30) et l'élément intérieur (40),
où la portion cylindrique s'étendant de l'élément extérieur (30) forme une section de support dure (33) qui entoure le périmètre de la zone de tétine (N) de l'utilisateur,
et la portion cylindrique s'étendant depuis l'élément intérieur (40) forme une section d'entourage, dont une extrémité frontale est formée en une section d'installation (43) pour buter contre un côté intérieur de l'élément extérieur (30),
et qui entoure aussi complètement, sans aucun espace, la zone de tétine (N), où l'élément intérieur comprend une section de butée déformable (45) qui se déforme élastiquement et qui est apte à établir un contact proche, sans aucun espace, avec un périmètre d'une zone de tétine (N) d'un utilisateur,
où une ouverture (41) qui relie l'espace intérieur (S2) entouré par la section d'entourage à l'extérieur est réalisée dans l'élément intérieur (40), et un trou traversant (31) qui est relié à l'ouverture (41) est réalisé dans l'élément extérieur (30).

2. Cache-tétine (20) selon la revendication 1, dans lequel la section d'installation (43) couvre la portion d'extrémité avant de la section de support (33).

3. Cache-tétine (20) selon l'une quelconque des revendications 1 ou 2, dans lequel la section d'entourage de l'élément intérieur (40) possède une section de paroi non interrompue (43), et comme continuation depuis la section de paroi (43), une section courbée (44) qui est courbée vers l'intérieur sur un côté intérieur de celle-ci est prévue, et comme continuations depuis la section courbée (44), la section de butée déformable (45) qui est en expansion vers l'extérieur, et une section de butée et de protection (46) qui est courbée vers l'extérieur sur un côté extérieur de la section de butée déformable (45) et couvre un bord extérieur de l'élément extérieur (30) sont réalisées.

4. Cache-tétine (20) selon la revendication 1, dans lequel une ouverture (42) d'une aire de surface plus grande que l'ouverture (41) ou le trou traversant (31) est formée dans une position de l'élément intérieur (40) sur l'extérieur de l'ouverture (41) et/ou une position de l'élément extérieur (30) sur l'extérieur du trou traversant (31) .
